Europäisches Patentamt

**European Patent Office**

Office européen des brevets

(19)

(11) Publication number : **0 506 862 B1**

(12) # EUROPEAN PATENT SPECIFICATION

(45) Date of publication of patent specification :
**09.11.94 Bulletin 94/45**

(51) Int. Cl.[5] : **G01N 33/68,** G01N 33/50

(21) Application number : **91902633.6**

(22) Date of filing : **21.12.90**

(86) International application number :
**PCT/US90/07613**

(87) International publication number :
**WO 91/10134 11.07.91 Gazette 91/15**

(54) GLUTAMATE AS A GROWTH DETERMINANT.

(30) Priority : **22.12.89 US 455539**

(43) Date of publication of application :
**07.10.92 Bulletin 92/41**

(45) Publication of the grant of the patent :
**09.11.94 Bulletin 94/45**

(84) Designated Contracting States :
**AT BE CH DE DK ES FR GB GR IT LI LU NL SE**

(56) References cited :
**ENDOCRINOLOGY,vol. 106, no. 1, 1980; B.S.
WONGET et al., pp. 268-274
METABOLISM,vol .38, no. 3, 1989; W.T.
DAHMS et al., pp. 197-203
AMERICAN JOURNAL OF PHYSIOLOGY, vol.
250, 1986; T.C. WELBOURNE et al.,
pp.E457-E463**

(56) References cited :
**AMERICAN JOURNAL OF PHYSIOLOGY, vol.
257, No. 6, 1989, Tomas Welbourne et al., page
E959-page E962
HORM. METABOL., vol. 16, 1984, A.M. Repellin
et al., page 539-page 543 and table 3**

(73) Proprietor : **GENENTECH, INC.
460 Point San Bruno Boulevard
South San Francisco California 94080 (US)**

(72) Inventor : **CRONIN, Michael, J.
615 Barriolhet
San Mateo, CA 94402 (US)**
Inventor : **WELBOURNE, Tomas, C.
4146 Maryland Avenue
Shreveport, LA 71106 (US)**

(74) Representative : **Nicholls, Kathryn Margaret et
al
MEWBURN ELLIS
York House
23 Kingsway
London WC2B 6HP (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

FIELD OF THE INVENTION

Methods are described for determining glutamate concentrations and ratios of glutamate with glutamine, alanine, urea and creatinine, as an indicator of a mammal's nitrogen metabolism, thereby facilitating growth optimizing therapy and disease treatment. The administration of glutamate in combination with a hormonal agent of nitrogen balance, or administration of glutamate alone to stimulate growth is further disclosed.

BACKGROUND OF THE INVENTION

Hormonal agents of nitrogen balance (HANB) such as growth hormone (GH) or insulin-like growth factor 1 (IGF-1), also known as somatomedin C (SM-C) are known to promote growth. For purposes of the present invention, GH will be discussed as representative of all hormonal agents of nitrogen balance.

The growth promoting effect of GH administration has long been associated with a positive nitrogen balance (Engel *et al.*, Metabolic actions of pituitary hormones. In: The Hormones, edited by G. Pincus *et al.*, New York: Acad. Press 1964m vol v, p. 69; Long, C.N.H., Ann. NY Acad. Sci. 43:383-426 [1943]) and reflected by decreased urea excretion (Russell, J.A., Am. J. Clin. Nutr. 5:404-416 [1957]; Souba *et al.*, Metabolism 34:450-456 [1985]). Recently it has been confirmed that this results from a true reduction in urea production (Dahms *et al.*, Metabolism 38:197-203 [1987]). Despite being the focus of numerous studies, the mechanisms underlying the shift of nitrogen from ureagenesis into growth processes are unknown. Therefore, there is a need for a method of determining what nitrogen carrier responds to growth promoting hormones. The traditional nitrogen carrier is glutamine. As will be seen, the present invention establishes that the activities of urea cycle enzymes, already present in great excess, are little affected by GH administration (McLean and Gurney, Biochem . J.87:96-104 [1963]; Palekar *et al.*, Biochem. Biophys. Res. Comm. 100:1604-1610 [1981]). This leads to the possibilities that either nitrogen flow from the periphery may be limiting (Haymond *et al.*, J. Clin. Endocrinol. Metab. 42:846-855 [1976]) or that hepatic uptake of these interorgan nitrogen carriers may be altered (Jefferson *et al.*, J. Biol. Chem. 250:197-204 [1975]). It is well known that muscle releases nitrogen into the blood predominantly in the form of glutamine and alanine (Ruderman and Berger, J. Biol. Chem. 249:5500-5506 [1974]) and that the liver exhibits well defined transporters, systems N and A (Kilberg *et al.*, J. Biol. Chem. 255:4011-4019 [1980]; Kilberg, M.S., J. Membr. Biol. 69:1-12 [1982]). In catabolic states for example, nitrogen flows from muscle largely in the form of glutamine to the gut (Souba *et al.*, Metabolism 34:450-456 [1985]; Welbourne, T.C., Metabolism 37:520-525 [1988]) and liver, with lesser nitrogen transported as alanine. In the liver the nitrogen ultimately supports accelerated ureagenesis (Souba *et al.*, Metabolism 34:450-456 [1985]; Welbourne, T.C., Metabolism 37:520-525 [1988]). Accordingly, GH may control this flow by either limiting peripheral release of these carriers or by nullifying hepatic net uptake of these carriers. Indeed, the latter might be brought about by the induction of hepatic glutamine synthetase (Wong and Dunn, Biochem. Biophys. Res. Commun. 79:876-884 [1977] and Wong et al, Endocrinol 106, 268-274 (1980)) and increased glutamine production at the expense of urea.

Glutamine As Nitrogen Carrier

The induction of hepatic glutamine synthetase by GH administered to a hypophysectomized rat is well documented (Wong and Dunn, Biochem. Biophys. Res. Commun. 79:876-884 [1977]) and can also be demonstrated in cultured liver cells (Gebhardt and Mecke, In: Glutamine Metabolism in Mammalian Tissues. Eds. Haussinger and Seis, Springer-Verlag Berlin Heidelberg, 1984 pp 103-112). This mechanism that could cause the flow of $NH_3$ into the high affinity glutamine synthetase pathway at the expense of the carbamoyl phosphate synthetase and thereby spare incorporation into urea. However, the failure to observe any change in net glutamine uptake argues against this possibility. Furthermore, for $NH_3$ to gain access to glutamine synthetase, some additional effect on altered $NH_3$ incorporation into urea must be postulated because glutamine hydrolysis occurs in the upstream periportal region (Haussinger, D., Eur. J. Biochem. 133:269-275 [1983]) and is closely coupled to ureagenesis (Buttrose *et al.*, Am. J. Physiol. 252:E746-E750 [1987]; Meijer, A.J., FEBS Lett. 919:249-251 [1985]). In contrast, glutamine synthesis occurs in the downstream perivenous region of the liver (Haussinger and Gerok, Eur. J. Biochem., 136:421-425 [1983]).

The present invention surprisingly reveals a mechanism for resolving this paradox: shunting of the glutamine nitrogen away from urea production via hepatic glutamate release. The liver's handling of glutamate is all the more remarkable because glutamate is avidly removed from the blood by the hypophysectomized rat liver. Not surprisingly, the arterial blood glutamate is depleted in hypophysectomized rats falling to only 20 percent of normal (Dahms *et al.*, Metabolism 38:197-203 [1987]), consistent with the liver's normal role as the major source of blood glutamate (Welbourne *et al.*, Am. J. Physiol. 250:E457-E463 [1986]). In addition, Welbourne et al, Am J Physiol 257,E959-E962 (1989), discloses the effect of grown hormone on hepatic glutamate handling in vivo

The prior art taught that acute administration of monosodium glutamate (MSG) in adult rats (1g/kg i.p.) caused an immediate, long-lasting suppression of GH secretion (Terry *et al*, Brain Research, 217:129-142 [1981]). Such suppression of GH secretion would lead those skilled in the art to avoid the use of glutamate to promote growth due to this effect. Contrary to the prior art, the present invention surprisingly finds that low doses of glutamate promote growth.

A recent 1989 review of hormonal agents of nitrogen balance in the kidney does not mention a relationship between glutamate flux and hormone response (Hammerman, Amer. J. Physio. 257:F503-514 [1989]). Methods for the determination of glutamate are well known in the field of biochemistry. Methods for detecting glutamate are exemplified by EPO application 87307126; Alfredsson *et al.*, J. Chromotog., 424:378-84,(1984); in urine, Matsuura *et al.* in Anal. Biochem., 117:259-65, (1981); and for glutamic transaminase activity in USP 4,086,142.

One object of the present invention is to provide an improved method for evaluating whether a person is deficient in a hormonal agent of nitrogen balance. Another objective to provide an assay to determine whether a patient is responsive to the therapeutic administration of a hormonal agent of nitrogen balance. Still another object of the present invention is to provide a method for assuring patient compliance with therapeutic use of a hormonal agent of nitrogen balance. Yet another application for the method of the present invention is to optimize the therapeutic dosage of a hormonal agent of nitrogen balance. Another object of the present invention is the use of glutamate to promote growth by the therapeutic administration of glutamate alone or in combination with a hormonal agent of nitrogen balance.

## SUMMARY OF THE INVENTION

The present invention describes a method for evaluating the nitrogen metabolizing status of an animal as an indication of a need for administration of a hormonal agent of nitrogen balance (HANB).

One embodiment of this method for diagnosing deficiency in a HANB in a patient comprises: a) providing a sample body fluid or tissue; b) quantitating the molar concentration of glutamate and a second compound selected from glutamine, alanine, urea or creatinine in said sample; c) determining the molar ratio of said glutamate to said second compound; d) comparing said molar ratio with a standard molar ratio found in a sample of patients known not to have a deficiency in a hormonal agent of nitrogen balance, and if said molar ratio is statistically different from said standard molar ratio, then the diagnosis is a deficiency in a hormonal agent of nitrogen balance which may be treated by administering the HANB. The method may be applied to a body fluid is selected from the following: serum, blood, urine, sputum, mucus, amniotic fluid, perspiration, tears, saliva, spinal fluid, milk or plasma. The preferred HANB are growth hormone and insulin-like growth factor-1.

A preferred application of the method is where the bodily fluid is urine, the second compound is glutamine and the standard molar ratio for glutamine/glutamate is less than 10. Another application of the method is when the bodily fluid is urine, the second compound is arginine and the standard molar ratio for alanine/glutamate is less than 5. Still another application of the method is where the bodily fluid is urine, the second compound is creatinine and the standard molar ratio is replaced by a standard mole/weight ratio for glutamate/creatinine which is greater than 150 nmol/mg creatinine.

The method of the present invention has application for determining the dosage of a HANB in a patient where the method comprises: (a) obtaining a sample of urine from the patient prior to the administration of the HANB; (b) determining the ratio of glutamate to a second compound selected from the group of glutamine, alanine, urea and creatinine, or the reciprocal thereof, in the urine sample of step (a); (c) administering a test dose of HANB to the patient: (d) collecting a sample of urine from the patient; (e) determining the ratio of glutamate to said second compound selected from the group of glutamine, alanine, urea and creatinine, or the reciprocal thereof, in the urine sample of part (d), provided that the ratio is calculated on the same basis or reciprocal as was used in part (b), and (f) determining the difference between the ratio of step (b) and step (e), and if the difference is statistically significant, adjusting the dosage of the HANB up or down as required until the difference between step (b) and step (e) is not statistically significant. When the method above uses the ratio of glutamate to glutamine, and if the ratio determined in step (b) substantially exceeds that which is determined in step (d) then the patients HANB dosage is increased. However, if the ratio determined in step (b) is substantially less than that which is determined in step (d), then the patients HANB dosage is decreased. When the ratio determined in step (b) is substantially the same as that which is determined in step (d), the patient's HANB dosage is maintained because that is the optimum dosage. This method for determining the dosage of a HANB may be applied to human growth hormone or insulin-like growth factor-1. Moreover, the method may be applied when the HANB is human growth hormone or growth hormone plus growth hormone binding protein. While the method may be applied to adults and children, in a preferred embodiment, the method is applied to patients younger than 20 years of age.

Another embodiment of the method of the present invention has application in monitoring the metabolic condition of a patient receiving a HANB. This monitoring method comprises: (a) obtaining a sample of urine from the patient prior to the therapeutic administration of the HANB; (b) determining the ratio of glutamate to a second compound selected from the group of glutamine, alanine, urea and creatinine, or the reciprocal thereof, in the urine sample of step (a); (c) administering the therapeutic dose of HANB to the patient: (d) collecting a sample of urine from the patient; and, (e) determining the ratio of glutamate to the second compound selected from the group of glutamine, alanine, urea and creatinine, or the reciprocal thereof, in the urine sample of part (d), provided that the ratio is calculated on the same basis or reciprocal as was used in step (b). This method may be applied when the ratio determined is that of glutamate to glutamine, and the HANB is a growth hormone, an insulin-like growth factor-1, human growth hormone, human growth hormone plus growth hormone binding protein, insulin-like growth factor-1 or insulin-like growth factor-1 plus insulin-like growth factor binding protein, human insulin-like growth factor-1 or human insulin-like growth factor-1 plus human insulin-like growth factor binding protein.

The method of the present invention has application when deficiency in a HANB in a patient follows treatment with one of the following: surgery, radiation, chemotherapy, antibiotics, hormone agonists, hormone antagonists, hormone inhibitors, antibodies, or nutritional supplements.

A preferred application of the method of the present invention is in a method for diagnosing deficiency in a HANB in a patient which comprises: a) providing a urine sample; b) quantitating the concentration of glutamate in the sample; c) if the concentration of glutamate in said sample is greater than 150 nmol/mg creatinine, then administering a therapeutic amount of a HANB. Another preferred method for diagnosing deficiency in a HANB in a patient is: a) providing a urine sample; b) quantitating the concentration of glutamate and glutamine in the sample; c) determining the ratio of the glutamine divided by the glutamate; d) if the ratio is less than 10, then administering a therapeutic amount of a HANB. These methods may be applied where the HANB is selected from growth hormone or insulin-like growth factor-1.

The surprising observation that glutamate serves as a major nitrogen carrier maintaining nitrogen balance resulted not only in the methods of measuring responses to a HANB but also the discovery that glutamate may be used to promote growth and maintain nitrogen balance in a mammal deficient in a HANB. This method of promoting growth and nitrogen balance comprises administering a therapeutically effective amount of glutamate to promote growth and nitrogen balance. The therapeutically effective amount of glutamate may be administered by a route selected from the following: oral, topical, parenteral injection such as intramuscular injection, subcutaneous injection or intravenous injection; suppository, or inhalation. The dosage of glutamate administered may be a dosage of 1.0 to 500 mg/kg/day, more preferably 10 to 250 mg/kg/day, and most preferably 50 to 150 mg/kg/day.

DESCRIPTION OF THE FIGURES

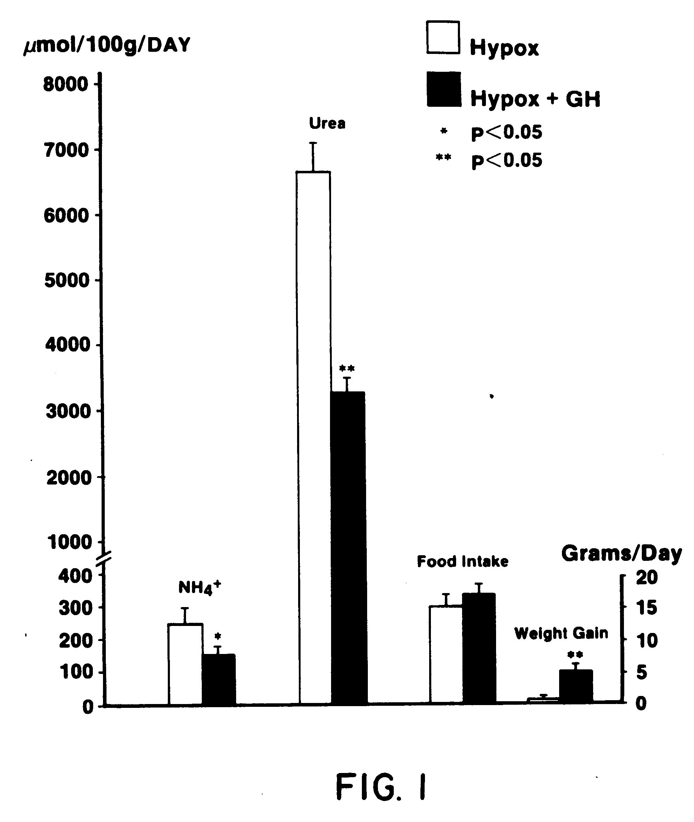

Figure 1. Effect of GH on urinary ammonium, ($NH_4^+$), and urea excretion by 1) hypophysectomized, (hypox), and 2) GH (GH) supplemented, (hypox + GH) rats. GH at 100µg/100g BW or vehicle alone were administered to animals placed into metabolic cages for 5 days followed by 3 additional days during which daily food consumption, body weight and urine excretions were measured. Results are means ± S.E. from the 3 day average for 6 rats per group.

DESCRIPTION OF THE PREFERRED EMBODIMENTS

The methods of the present invention may be used to determine the status of a mammal's nitrogen metabolism as a measure of deficiency in a HANB, or as a measure of responsiveness to a HANB. This determination is accomplished by 1) measuring the molar concentration of glutamate and the molar concentration of a second compound selected from glutamine, alanine, urea or creatinine in a sample of bodily fluid; 2) determining the molar ratio of said glutamate to said second compound; and 3) comparing said molar ratio with a standard molar ratio found in a sample of patients known not to have a deficiency in a HANB; and if said molar ratio exceeds said standard molar ratio, then the diagnosis is a deficiency in a HANB. The hormonal agents of nitrogen balance causing the deficiency may be determined by direct measurement for known hormonal agents of nitrogen balance. These methods may also be used to optimize the dosage of a HANB and to detect nitrogen imbalance resulting from the presence of disease conditions.

4

Measurements of glutamate, glutamine, alanine, creatinine and urea may be made in any bodily fluid, or in any tissue extract. Among the body fluid are serum, blood, urine, sputum, mucus, amnionic fluid, perspiration, tears, saliva, spinal fluid, milk, semen or plasma. One preferred bodily fluid for convenient assay is urine. A second preferred bodily fluid is blood, plasma or serum. The preferred measurements is of glutamate plus a second compound selected from glutamine, alanine, creatinine or urea. The calculated glutamate ratios may be expressed with glutamate in the numerator or denominator of the ratio, and both ratios are diagnostically equivalent indicators of the ratios. These ratios, with glutamate in the numerator or in the denominator, are anticipated to be equally useful depending which is most visually appealing. All ratios compared must be calculated using glutamate in the same mathmatical relationship with the second compound.

The first approach used to evaluate the effect of GH on serum carriers of nitrogen led to a surprising result. This first approach was to monitor both the delivered glutamine load and the liver's extraction of glutamine in the growth-arrested and growth-accelerated states. These measurements permitted a determination of whether glutamine concentration changed as predicted on the organ level. Surprisingly, neither the delivered glutamine load nor the uptake by the liver were reduced with GH treatment despite a depressed ureagenesis. We concluded that glutamine was not the nitrogen carrier responding to GH (see Example 1).

However, the present invention showed for the first time that the hepatic glutamate concentration was dramatically affected by GH administration. Administration of GH caused a reversal in the function of the liver from an avid uptake organ for glutamate to an enormous release organ for glutamate. The present invention shows that this unexpected and surprising change in glutamate concentration is a major metabolic mechanism underlying physiological and developmental nitrogen homeostasis, and a critical cofactor in the promotion of growth. The present invention teaches for the first time that GH treatment restores arterial glutamate levels by reducing this liver uptake and returning the liver's role to an exporter of glutamate. However, this rate of glutamate release is some 4-fold higher than what has been normally observed (Welbourne et al., Am. J. Physiol. 250:E457-E463 [1986]), Since arterial levels are stable, this indicates that a large removal of glutamate is occurring at some site or sites located in the periphery. Presumably, it is this interorgan relationship involving glutamate flow that ultimately results in the redistribution of nitrogen from ureagenesis into growth related processes. Interestingly, blood glutamate is greatly depressed in hypopituitary dwarfs and restored by GH administration (Sato and Uchigata, Acta Endocrinol. 92:398-405 [1979]).

Determination of Indicator Ratios

Preferred methods for determining the growth status of a mammal are to measure the following: 1) glutamate to glutamine ratio; and 2) glutamate to alanine ratio. These determinations may be made on any bodily fluid or tissue. Among the preferred tissues are muscle, mucus membrane, skin, kidney and liver. These indicator ratio changes following the administration of a HANB occur because glutamate transport from the liver into growth responsive cells is accelerated while the transport of glutamine is not accelerated. This response to GH accounts for the rise in the glutamate/glutamine ratio coming out of the liver and the fall in the glutamate/glutamine (or alanine) ratio in the urine. GH promotes uptake of glutamate by the kidneys thus decreasing the glutamate in urine. These glutamate ratios measured in the target bodily fluid, are measured both pre and post administration of a HANB, thus indicating responsiveness to the administered HANB.

The physiological nitrogen balancing response to a HANB such as GH is related to these ratios because glutamate released by the liver becomes the major if not the unique vehicle for nitrogen redistribution at the expense of urea. Therefore, a fall in the urine glutamate/glutamine ratio precedes any detectable change in urea since the pool size of the urea is very large and contributing sources are multiple and highly variable.

The approximate limits for the glutamate/creatinine ratios in GH deficient mammals are greater than 150 nmol/mg creatinine and greater than 3,000 nmol/mg creatinine for urine and venous plasma, respectively. The administration of GH within two hours decreases these ratios to below 30 nmol/mg creatinine for urine and below 2,000 nmol/mg creatinine for venous plasma. Two preferred criteria are available for determining GH responsiveness in slow growers. The first criteria for slow growers is urinary glutamate (Glu) per mg creatinine (Glu/creatinine) in the range of about greater than 150 nmol/mg creatinine and a glutamine/glutamate (Gln/Glu) molar ratio in the range of about less than 10.

Normals (non-slow growers) have similar glutamate ratio criteria in the range of about "less than 90 nmol/mg creatinine" for Glu/creatinine; and "more than 15" for Gln/Glu molar ratios. A patient is considered responsive to GH when the urinary values for glutamate are about 20-60 nmol/mg creatinine for Glu and about 17-40 for Gln/Glu molar ratios. It is noted that there seems to be special significance to the Gln/Glu value, since forearm blood exhibits a ratio of less than 17 for slow growers and greater than 27 after GH administration.

Therefore, these two ratios provide an example of a method of evaluating the therapeutic effect of growth promoting hormones such as GH and IGF-1 (somatomedin C). These indices provide such an evaluation before, during and after hormone administration. The noninvasive monitoring of urine constituents is particularly appealing and allows for the convenient monitoring of hormone response time and response duration, thereby providing valuable criteria for establishing dose, pattern and frequency of hormonal agent administration.

Multiple ratios may also be used in the method of the present invention. These include (glutamate/glutamine); (glutamate/alanine); (glutamate/urea) or (glutamate/creatinine) ratios and their reciprocates. Ratios other than (glutamate/glutamine) may be preferred in particular disease or metabolic states where the glutamine value is less reliable as a control value. In measuring glutamate in urine, it is important to acidify the urine immediately upon collection to prevent glutamine deamination which may result in an erroneous measurement of both glutamine and glutamate, thereby resulting in an even more erroneous glutamate/glutamine molar ratio.

For convenience in intrepreting molar ratio results, a standard curve, for example of glutamate/glutamine ratio vs GH activity, that indicates the activity present of a HANB can be constructed using methods well known to the science of pharmacology as described in texts such as Goodman and Gilman's The Pharmacological Basis of Therapeutics, sixth edition, Macmillan Publishing Co. Inc.(1980), especially sections I and XV. The construction of such a standard curve can be prepared for any of the glutamate containing molar ratios using a defined patient population having the desired clinical condition and metabolic status. For example, growth deficient children, gigantism, acromegaly, kidney disease, liver disease, malignancy, psychosocial stress, under-nutrition, pulmonary insufficiency, trauma, infection, gastrointestinal disorder, osteoporosis, aged, cancer, bedridden or diabetes.

The methods of the present invention may also be used to detect the production of excessive amounts of a HANB, for example, in gigantism or in acromegaly. Following detection using the methods of the present invention, therapies can be used to reduce the disease condition while using the present invention's methods to monitor the response to therapy. Among the anticipated therapies are surgery, radiation, chemotherapy, and hormone antagonists or inhibitors such as antibodies.

## Significance of Test Ratios

The assay methods of the present invention facilitate a quick and easy determination of the metabolic nitrogen status of the subject, presumptive deficiency in HANB and the monitoring of responsiveness to administration of a HANB. The types of disease or deficiency states where this method finds use are many. Included are catabolic states resulting from trauma, sepsis, chronic renal and liver failure, malignancy, psychological stress, wasting, cancer and malnutrition. The administration of a hormonal agent of nitrogen balance (such as growth hormone) is believed to be caused by an uncoupling of the glutamine nitrogen flow from tissue protein into urea. This uncoupling is accomplished by shunting the nitrogen away from urea production in the liver and into glutamate production. This glutamate is released into the blood and transported into the cells where it acts as a growth promoting cofactor. While this is believed to be the underlying mechanism, the present invention is not dependent upon such a methanism or theory, nor is such a theory necessary to practice the invention.

The monitoring of the glutamate/glutamine ratio in biopsied muscle and/or liver can also provide an index of growth promoting hormone effectiveness. When monitoring GH, it is expected to find increased liver, muscle and kidney glutamate levels in the tissue. However, the only increase in plasma glutamate is found leaving the liver. When monitoring IGF-1 (somatomedin C) it is expected to find increased muscle glutamate. In the absence of biopsy material, an elevated venous red cell glutamate to plasma glutamate ratio provides a less specific index (for GH and IGF-1). In the absence of blood sampling, urinary ratios of glutamate/glutamine may be conveniently used, corrected for urine production rate by measuring urinary creatinine. Failure of the GH response is confirmed by high urea/creatinine ratios in urine and low liver and plasma glutamate levels. Failure of the IGF-1 (SM-C) response is shown by high circulating plasma glutamate, low muscle glutamate, and high liver glutamate. Therefore, the therapeutic use of GH or IGF-1, with or without serum binding proteins, can be monitored as to its efficacy in preventing nitrogen wasting (as with GH); and effecting reincorporation of nitrogen into protein synthesis via glutamate recycling (in the case of IGF-1).

The initial reason for developing the assay methods of the present invention was to determine if the well known effect of GH on whole body nitrogen balance could be discerned in terms of hepatic glutamine balance. Specifically, could the depressed urea production be related to either a diminished delivered load of glutamine precursor or to a modulation of net glutamine uptake. The present invention shows that neither the availability of glutamine, nor alanine for that matter, limits urea production in these hypophysectomized animals receiving GH. Thus, the effect of GH on urea production differs from the intact acidotic and starved animals in which arterial glutamine levels fall, associated with a large renal extraction and elevated ammonia production (2,22,24). More surprising was the failure of GH to alter net glutamine uptake, either by blocking unidirectional glutamine uptake or by accelerating its rate of synthesis and thereby counter-balancing uptake.

Definitions:

A "nitrogen balancing therapy" for purposes of the present invention is any therapy, such as administering a HANB, such as GH, IGF-1, growth hormone binding protein, insulin-like growth factor binding protein (5 known); glutamate or any therapeutically effective agent, that causes the level of glutamate to achieve the concentration needed to promote nitrogen balance.

A "hormonal agent(s) of nitrogen balance" (HANB) includes hormones and cofactors controlling or modulating nitrogen in the body as indicated by the presence of glutamate, glutamine, alanine, creatinine, and urea. Among the HANB are hormones such as GH, IGF-1, GH binding protein, IGF-1 binding proteins, combinations of these hormones and binding proteins; and glutamate as a cofactor and as a nitrogen source.

"Nitrogen balance" is defined as a metabolic process by which the animal body preserves protein to promote survival. The protein preserved includes, but is not limited to, muscle, organ tissue, enzymes, connective tissue, blood components, cell structural protein and bone.

"Glutamate" as used includes both glutamic acid and salts of glutamate, such as monosodium glutmate (MSG). Included are all physiological acceptable salts of glutamate including but not limited to sodium and potassium.

A "statistically different molar ratio" refers to a difference of at lease one standard deviation away from the standard molar ratio, and more preferably 1.5 standard deviations, and most preferably 2 or more standard deviations away from the standard molar ratio. Statistical analysis is performed applying a normal Gaussian distribution. For a single patient and single sample, transverse analysis is applied. For a single patient and multiple samples, a longitudinal analysis is applied.

Glutamate Metabolism

The production of glutamate in response to the administration of an HANB most likely is under the control of glutamate transport. However, glutamate dehydrogenase also plays a substantial facilitative role in glutamate's metabolism. Ammonia nitrogen generated from glutamine via hepatic glutaminase is directed through L-glutamate dehydrogenase which, in animal tissues, can utilize either NAD or NADP. Glutamate dehydrogenase is a regulatory enzyme having a number of allosteric effectors whose complex effects depend on many metabolic factors. For example, ADP may be either stimulatory or inhibitory, depending on the NAD concentration, whereas GTP is strongly inhibitory to the enzyme. Glutamate formed from glutamine intramitochondrially can be exported via the hydroxyl ion/glutamate exchanger. As a consequence of GH's action to extrude protons, the remaining hydroxyl ions drive glutamate out of the mitochondria and away from ureagenesis thereby preserving glutamate. Glutamate uptake is driven at tissues not producing glutamate by an inward directed proton gradient. Because GH acts at these sites to expand this proton gradient, glutamate uptake occurs in tissues exhibiting such proton exchanges resulting in the capability to take up glutamate by transporters. Furthermore, glutamate uptake can be shown to modulate the uptake of other amino acids resulting in cellular protein synthesis and hypertrophy.

Administration of Glutamate

Given the central role of glutamate as a carrier of nitrogen to growing tissues and the stimulation of glutamate uptake by HANB, and in accordance with this invention, Glutamate is administered alone or in combination with HANB. The additional administered physiological level of glutamate supplements or, in glutamate insufficient individuals. The glutamate may supply the necessary physiological level of glutamate needed for nitrogen balance and growth. Glutamate itself may function as a cofactor for promoting nitrogen balance and growth. Induced in high levels by growth promoting hormones, the increased level of glutamate alone may be sufficient to optimize growth based upon indigenous HANB. The administered glutamate may further optimize, maintain and promote nitrogen balance and growth beyond that induced by administered growth-promoting hormones and other HANB

Formulation

The most preferred route of administration of glutamate is oral, by liquid, tablet or powder. However other routes of administration such as parenteral injection (intramuscular injection, subcutaneous injection, intravenous injection), suppository, sustained release implants, or inhalation are included. Glutamate may also be formulated for topical administration using carriers well known to those of ordinary skill.

The glutamate to be used for injection must be sterile. Sterility is readily accomplished by filtration through sterile filtration membranes (e.g., 0.2 μm membranes). The glutamate ordinarily will be stored in lyophilized form or as an aqueous solution if it is highly stable to thermal and oxidative denaturation.

If the glutamate is to be used parenterally, therapeutic compositions containing the glutamate generally are placed into a container having a sterile access port, for example, an intravenous solution bag or vial having a stopper pierceable by a hypodermic injection needle.

Sustained release formulations may also be prepared, and include the formation of microcapsular particles and implantable articles. For preparing sustained-release glutamate compositions, the glutamate is preferably incorporated into a biodegradable matrix or microcapsule. A suitable material for this purpose is a polylactide, although other polymers of poly-(a-hydroxycarboxylic acids), such as poly-D-(-)-3- hydroxybutyric acid (EP 133,988A), can be used. Other biodegradable polymers include poly(lactones), poly(acetals), poly(orthoesters), or poly(orthocarbonates). The initial consideration here must be that the carrier itself, or its degradation products, is nontoxic in the target tissue and will not further aggravate the condition. This can be determined by routine screening in animal models of the target disorder or, if such models are unavailable, in normal animals. For examples of sustained release compositions, see U.S. Patent No. 3,773,919, EP 58,481A, U.S. Patent No. 3,887,699, EP 158,277A, Canadian Patent No. 1176565, U. Sidman et al., "Biopolymers" 22:547 [1983], and R. Langer et al., "Chem. Tech." 12:98 [1982].

For obtaining a gel formulation, the glutamate formulated in a liquid composition may be mixed with an effective amount of a water-soluble polysaccharide or synthetic polymer such as polyethylene glycol to form a gel of the proper viscosity to be applied topically. The polysaccharide that may be used includes, for example, cellulose derivatives such as etherified cellulose derivatives, including alkyl celluloses, hydroxyalkyl celluloses, and alkylhydroxyalkyl celluloses, for example, methylcellulose, hydroxyethyl cellulose, carboxymethyl cellulose, hydroxypropyl methylcellulose, and hydroxypropyl cellulose; starch and fractionated starch; agar; alginic acid and alginates; gum arabic; pullulan; agarose; carrageenan; dextrans; dextrins; fructans; inulin; mannans; xylans; arabinans; chitosans; glycogens; glucans; and synthetic biopolymers; as well as gums such as xanthan gum; guar gum; locust bean gum; gum arabic; tragacanth gum; and karaya gum; and derivatives and mixtures thereof. The preferred gelling agent herein is one that is inert to biological systems, nontoxic, simple to prepare, and not too runny or viscous, and will not destabilize the glutamate held within it.

Preferably the polysaccharide is an etherified cellulose derivative, more preferably one that is well defined, purified, and listed in USP, e.g., methylcellulose and the hydroxyalkyl cellulose derivatives, such as hydroxypropyl cellulose, hydroxyethyl cellulose, and hydroxypropyl methylcellulose. Most preferred herein is methylcellulose.

The polyethylene glycol useful for gelling is typically a mixture of low and high molecular weight polyethylene glycols to obtain the proper viscosity. For example, a mixture of a polyethylene glycol of molecular weight 400-600 with one of molecular weight 1500 would be effective for this purpose when mixed in the proper ratio to obtain a paste.

The term "water soluble" as applied to the polysaccharides and polyethylene glycols is meant to include colloidal solutions and dispersions. In general, the solubility of the cellulose derivatives is determined by the degree of substitution of ether groups, and the stabilizing derivatives useful herein should have a sufficient quantity of such ether groups per anhydroglucose unit in the cellulose chain to render the derivatives water soluble. A degree of ether substitution of at least 0.35 ether groups per anhydroglucose unit is generally sufficient. Additionally, the cellulose derivatives may be in the form of alkali metal salts, for example, the Li, Na, K, or Cs salts. If methylcellulose is employed in the gel, preferably it comprises about 2-5%, more preferably about 3%, of the gel and the glutamate is present in an amount of about 300-1000 μg per ml of gel.

The dosage of glutamate to be employed is dependent upon the factors described above, especially the type of growth deficiency or nitrogen balance being treated. As a general proposition, a dose of about 1.0 to 500 mg/kg of glutamate may be administered to the patient, whether orally delivered or via, e.g., multiple single administrations, continuous infusion, or bolus slow release injection. For example, an initial dose of the glutamate is administered to the patient by injection or infusion. For repeated administrations over several days or longer, depending on the condition, the treatment is repeated until a desired growth occurs. However, other dosage regimens may be useful. The most preferred route is oral administration in solution, tablets or powders, alone or in combination with carriers such as food. The preferred oral dose is in the range of 1.0 to 800 mg/kg/day, more preferably the range of 10 to 400 mg/kg/day, and most preferably 50 to 150 mg/kg/day. Glutamate may be administered through out the day, in singular or multiple oral doses to achieve the final dosage.

According to another embodiment of the invention, the effectiveness of the glutamate may be improved by administering it serially or in combination with another agent that is effective for this purpose, such as one or more hormonal agent of nitrogen balance such as GH or IGF-1 or their respective serum binding proteins. Such other agents may be present in the composition being administered or may be administered separately. Growth-promoting hormones, methods of making and their methods of use are described in US Patents 4,755,465; 4,342,832; 4,634,677; 4,604,359; 4,601,980; 4,658,021; 4,898,830; and Sara *et al*, Physiological Reviews, 70:591-614 (1990) and Daughaday *et al*, Endocrine Reviews 10: 68-91 (1989). Similarly, agents known to promote the activity of a hormonal agent of nitrogen balance, such as GH, IGF-1, growth hormone binding protein (Herington, A.C. *et al.* J. Clin. Invest. 77:1817-1823 [1986]) and insulin-like growth factor-1 binding proteins (Pekonen *et al.*, J. Immunoassay 10:325-327 [1989]) may be administered in combination with glutamate.

The following examples illustrate the methods of the present invention, but should not be construed to limit the invention.

EXAMPLE 1

Effect of Growth Hormone on Rat Liver Nitrogen Metabolism Experimental Animals

Growth hormone experiments were performed on hypophysectomized male Sprague Dawley rats weighing 250 to 300 grams (Harlan, Indianapolis, IN) and maintained in metabolic cages on 5 percent dextrose and Purina rat chow ad libitum. Each lot of animals was divided into a control and GH supplemented group: the latter received methionyl human GH (Genentech, San Francisco, CA) dissolved in 0.15M NaCl and injected intramuscularly daily; controls received the vehicle. The responsiveness of the hypophysectomized animals was determined by following daily body weights, food intake and urinary urea and ammonium excretion rates (Welbourne *et al.*, Am. J. Physiol. 250:E457-E463 [1986]).

Hepatic Balance Measurements:

To measure net uptake and release of metabolites across the intact liver it is necessary to simultaneously determine both blood flows and, in addition, the respective arteriovenous concentration differences. This was accomplished in both hypophysectomized and GH treated hypophysectomized rats as detailed below. The animals were anesthetized with inactin, 120 mg/kg, and placed on a heated animal board; core temperature was carefully maintained at 37°C throughout the study, employing a heating lamp when necessary. A PE 240 tubing was inserted into the trachea to ensure patency of the airway followed by cannulation of the jugular vein through which 0.15M NaCl containing 1 percent mannitol and bromosulphalein (BSP) was infused at the rate of 20 $\mu$l/min/100g; BSP concentration was calculated to deliver 150 $\mu$g/min/100g BW. Following cannulation of the carotid artery for arterial samples, the abdomen was opened by a midline incision and 24 gauge needles attached to silastic tubing implanted into the portal and hepatic veins as previously described (Buttrose *et al.*, Am. J. Physiol. 252:E746-E750 [1987]; Welbourne *et al.*, Am. J. Physiol. 250:E457-E463 [1986]). A segment of the small intestine was laid out over a warm saline moistened gauze and a 30 gauge needle connected in an infusion line was then inserted into a tertiary branch of the mesenteric vein through which C-14 labelled paraamino-hippurate (p-[glycl-1-$^{14}$C] aminohippurate, 41 mci/nmol., New England Nuclear, Boston, MA) was infused at a rate of 0.003 $\mu$ci/min/100g BW in the above saline solution delivered at a volume flow rate of 20 $\mu$l/min/100g BW. After 45 minutes for equilibration, a single set of blood samples, 0.2 ml, were simultaneously drawn from the hepatic and portal veins and the carotid artery. These blood samples were promptly centrifuged at 10,000 RPM (0-4°C) and aliquots taken of plasma for the measurement of BSP by colorimetric analysis (Welbourne *et al.*, Am. J. Physiol. 250:E457-E463 [1986]), glutamine, glutamate and alanine by HPLC (24), urea by enzymatic analysis (Welbourne, T.C., Am. J. Physiol. 256:F1027-F1033 (1989]) and C-14 PAH by liquid scintillation spectrometry. Recovery of glutamine and glutamate standards, 50 nmol, added to plasma aliquots were 97.3 ± 1.5 and 87.8 ± 5.3 percent, respectively, N=3. Repetitive analysis of arterial samples gave a coefficient of variation of 3.6 and 2.2 percent for glutamine and glutamate, respectively.

Calculation of Hepatic Uptake and Release

Net uptake and/or release rates of glutamine, glutamate, alanine and urea were determined from the difference between the metabolite load entering and leaving the liver. These, in turn, were measured from their respective plasma flows time the arterial., portal and hepatic venous concentrations. Hepatic venous plasma flow (HPVF) was obtained by averaging the values of plasma flow measured by the BSP and PAH methods; although a strong correlation exists (Welbourne *et al.*, Am. J. Physiol. 250:E457-E463 [1986]) radiolabelled PAH concentration tends to be overdiluted at high flows and hence would be subject to greater error. Averaging the 2 methods, therefore, gave a more reliable estimate of the flow. Portal drained viscera plasma flow was determined by PAH dilution (Welbourne *et al.*, Am. J. Physiol. 250:E457-E463 [1986]). The difference between the HPVF and PVPF was used to obtain the hepatic arterial plasma flow. Uptake and release rates are expressed in nmol/min/100g BW of rat. Differences between the hypophysectomized and GH supplemented animals were judged significant at p=0.05.

Growth Hormone Restores Growth and Decreases Urea Excretion

Figure 1 shows 24h urea excretion under conditions in which liver net uptake will be determined. As expected, urea excretion falls about 50 percent, from $6780 \pm 760$ to $3210 \pm 270$ μmol/100g. This drop in urea excretion was accompanied by a drop in urinary ammonium nitrogen from $238 \pm 52$ to $145 \pm 19$ μmol/100g. Clearly this indicates a change in nitrogen retention since food intake was unchanged ($15 \pm 2$ versus $17 \pm 1$ grams/day). The GH treated animals gained some $5 \pm 1$ grams of body weight per day in contrast to the growth arrested controls which gained little, if any, weight. These weight observations ensure that measurements of steady state hepatic balance are indeed relevant to the biological response occurring at this time.

## Table 1
### Arterial Urea and Metabolite Concentrations[1]

|          | Urea    | Glutamine | Glutamate | Alanine |
|----------|---------|-----------|-----------|---------|
| Hypox    | 8,803   | 349       | 29        | 270     |
|          | ± 940   | ± 37      | ± 5       | ± 14    |
| Hypox+GH | 4,890   | 407       | 154       | 252     |
|          | ± 480   | ± 59      | ± 41      | ± 49    |
| P        | <0.01   | NS        | <0.01     | NS      |

[1]Values are means ± S.E. in mM for 6 animals per group. Arterial blood samples obtained from animals in Figure 1 under inactin anesthesia, processed and promptly analyzed as described. Statistics were performed with a Student's T-test.

Arterial levels of urea, glutamine, glutamate and alanine are presented in Table 1. In line with the decreased urea excretion, arterial urea concentration is significantly reduced with GH treatment, shifting from $8,830 \pm 940$ to $4,890 \pm 480$ μM, a 44 percent decrease (p 0.01).

Hepatic Glutamine Balance is Unchanged by Growth Hormone

The handling of arterial and portal venous glutamine loads by hypophysectomized and GH treated rats is shown in Table 2. Blood flow to the liver was unchanged by GH administration as was the contribution from arterial and portal inflows. The load of glutamine delivered to the liver was significantly increased with GH administration going from $2567 \pm 432$ to $1713 \pm 196$ nmol/min/100g, a 50 percent increase, (p 0.05). The hepatic arterial load was greater with GH than control treatment going from $1012 \pm 186$ to $1632 \pm 280$ nmol/min/100g, a 61 percent increase in glutamine load(p 0.05). However, the net liver uptake of glutamine was essentially unchanged, $494 \pm 135$ versus $342 \pm 110$ nmol/min/100g. Fractional removal of this glutamine load was also the same in the 2 groups, $19 \pm 4$ and $20 \pm 3$ percent, respectively, indicating that neither the glutamine load delivered to the liver nor the ability to take glutamine up are altered by GH treatment. Glutamine metabolism is therefore not the GH-responsive nitrogen carrier as anticipated. Some other carrier must be responsible for carrying the nitrogen which is no longer going into urea.

## Table 2

### Effect of Growth Hormone Administration on Hepatic Glutamine Uptake

| | HVPF[1] | PVPF ml/min 100g/ BW | HAPF | IN[2] | OUT nmol/ min/ 100g | NET UPTAKE |
|---|---|---|---|---|---|---|
| Hypox | 5.8 ± 0.3 | 2.9 ± 0.3 | 2.8 ± 0.4 | 1713 ± 196 | 1371 ± 198 | 342 ± 110 |
| Hypox+GH | 7.1 ± 0.7 | 3.0 ± 0.5 | 4.1 ± 0.7 | 2567 ± 432 | 2073 ± 364 | 494 ± 135 |
| P | NS | NS | NS | <0.05 | NS | NS |

[1]HVPF, PVPF and HAPF are hepatic and portal venous plasma flows and hepatic arterial flows respectively. [2]In is the sum of arterial and portal venous loads and out is the hepatic venous load. Net is the difference between inflow and outflow. Values are means ± S.E. in mM for 6 animals per group. Statistics were performed with a Student's T-test.

Hepatic Glutamate Balance Reverses from Uptake to Release

The handling of glutamate is remarkably different in the 2 experimental groups. Hypophysectomized rat livers remove substantial amounts of the glutamate load (71 ± 15 nmol/min/100g) despite the small delivered load of only 192 ± 20 nmol/min/100g. It should be noted that the liver is the major source of blood glutamate in the intact rat, releasing some 224 ± 37 nmol/min/100g (23). Thus, hypophysectomy results in the liver's reversal from a net producer of glutamate to a net consumer. In marked contrast, the liver of GH treated hypophysectomized rats exhibits a large net release of glutamate, 960 ± 229 nmol/min/100g despite being presented with a glutamate load almost 6 times the load delivered to hypophysectomized rat livers. This effect of GH supplement is also seen when the whole blood is analyzed and is of even greater magnitude. The hypophysectomized rat livers removed 220 ± 27 nmol/min/100g (N=3) with the GH supplement (p=0.001). The administration of GH resulted in a 1,893 nmol/min/ 100g increase in available glutamate.

## Table 3
### Effect of Growth Hormone Administration on Hepatic Glutamate Uptake

| | HVPF | PVPF ml/min 100g/ BW | HAPF | IN | OUT nmol/ min/ 100g | NET UPTAKE |
|---|---|---|---|---|---|---|
| Hypox | 5.8 ± 0.3 | 2.9 ± 0.3 | 2.8 ± 0.4 | 192 ± 20 | 121 ± 26 | 71 ± 15 |
| Hypox+GH | 7.1 ± 0.7 | 3.0 ± 0.5 | 4.1 ±0.7 | 1100 ± 327 | 2061 ± 550 | -960 ± 229 |
| P | NS | NS | NS | <0.01 | <0.005 | <0.001 |

Values are means ± S.E. in mM for 6 animals per group. Arterial blood samples obtained from animals in under inactin anesthesia, processed and promptly analyzed as described. Statistics were performed with a Student's T-test.

EXAMPLE 2

Hepatic Balance for Glutamine. Glutamate and Alanine in Relation to Urea Release in Rat

The relationship between glutamine, glutamate and alanine uptake and urea release is depicted in Table 4. GH administration reduced hepatic urea release from 3,146 ± 432 to 1,954 ± 320 nmol/min/100g (p=0.05), in line with both the 24 hour excretion level in Figure 1, and fall in the arterial urea concentration in Table 1. Despite this, glutamine uptake, either in absolute amounts or as fractional extraction, was unchanged; note that this uptake is approximately twice the rate of intact rats (Welbourne et al., Am. J. Physiol. 250:E457-E463 [1986]). Clearly, the depressed urea production is not dependent upon eliminating glutamine uptake. Alanine, another ureagenic precursor, had a slightly but not significantly reduced uptake with fractional extraction reduced from 37 ± 6 to 25 ± 4 percent (p=0.10). Glutamate uptake, 37 percent of the load, reverts to net release, which accounts for a sparing of some 1,031 (plasma) to 1,893 (whole blood) nmol/min/100g, or approximately the nitrogen shifted from release as urea.

## Table 4

### Effect of Growth Hormone Administration on Hepatic Glutamine, Glutamate, Alanine and Urea Handling

| | Glutamine[1] | Glutamate | Alanine[2] | Urea[2] |
|---|---|---|---|---|
| Hypox | 342 ± 110 | 71 ± 15 | 522 ± 120 | 3146 ± 432 |
| Hypox+GH | 494 ± 135 | -960 ± 229 | 363 ± 109 | 1954 ± 320 |
| P | NS | <0.001 | NS | <0.05 |

[1]Results are means ± S.E. in nmol/min/100g BW. [2]Calculated as described for glutamine and glutamate in Example 1. Six animal in each group.

EXAMPLE 3

Growth Hormone Depresses Splanchnic Glutamine Uptake and Elevates Glutamate Release in Dwarf Rats

Dwarf (dw/dw) rats have low pituitary and circulating GH levels resulting in growth retardation. Normal postnatal body growth in rats requires a redistribution of nitrogen from ureagenesis into protein synthesis. We studied hepatic uptake of the major nitrogen transporter glutamine in relation to urea release in these GH rats. Human recombinant GH (100 µg/100g body weight) was injected for six days into these female animals, producing a body weight gain from 136 ± 2 to 157 ± 3 gm (p=0.05). The vehicle control animals did not grow (134 ± 7 to 137 ± 8 gm). GH decreased urinary 24 h urea excretion (an index of production) from 2,770 ± 310 to 1950 ± 180 µmol/100g/day (p=0.05). To assess changes in fluxes of amino acids relevant to urea production, aortic and hepatic venous blood samples were drawn under ether anesthesia and the plasma concentrations of glutamine, glutamate and alanine were determined by HPLC. GH treatment decreased (p 0.05) arterial concentrations of all 3 amino acids (nmol/ml) [treatment vs control]: glutamine, 478 ± 17 vs 617 ± 14; glutamate, 24 ± 3 vs 40 ± 2; alanine, 240 ± 21 vs 346 ± 32. After GH treatment the arteriovenous (A-V) differences across the splanchnic bed plummeted from 262 ± 12 to 60 ± 28 nmol/ml for glutamine and from 90 ± 13 to -8 ± 34 nmol/ml for alanine, indicating a reduction of glutamine and alanine uptake. In contrast, A-V glutamate concentration changed from -2 ± 2 to a release of -13 ± 5 nmol/ml. The fall in hepatic ureagenesis, associated with reduced glutamine and alanine uptake and glutamate release, is consistent with an effect of GH on redistribution of nitrogen in the liver. In the dwarf rat, hepatic glutamate release, rather than peripheral glutamate uptake, appears to be limiting for growth. If true, the administration of exogenous glutamate should result in an increased growth rate.

To evaluate the effect of glutamate on growth in dwarf rats, four groups of 6 dwarf rats each, were treated as follows: 1) drinking water, 2) drinking water plus 0.1% glutamate, 3) growth hormone IM (100µg/100 g body weight) and 4) growth hormone IM (100µg/100 g body weight) plus drinking water with 0.1% glutamate. The rats consumed a daily average of 18 ml of the glutamate solution resulting in glutamate administration 18 mg of glutamate to give a dose of 100µM/100g/day. The dwarf rats have a limited ability to release glutamate in response to GH administration, therefore the oral administration of glutamate promotes some growth alone, and substantially stimulates growth in the presence of GH as shown in the table 5 below.

## Table 5

## Glutamate Stimulates Growth In Dwarf Rats

| Rat Group | Growth Response |
|---|---|
| water | 0.40 ±0.7 g/day |
| +0.1% Glu | 0.65 ±0.9 g/day |
| GH | 1.6 ±0.2 g/day |
| GH +0.1% Glu | 2.4 ±0.2 g/day |

The oral administration of 0.1% glutamate in drinking water induced growth in the dwarf rat. Water containing 0.1% glutamate plus IM growth hormone resulted in a 50% increase in weight gain in the dwarf rat. These results indicate that administration of glutamate, orally, or by other routes such as intramuscularly, subcutaneously, iv etc. will result in growth in mammals having insufficient glutamate availability.

EXAMPLE 4

Growth Hormone Enhances Rat Hindquarter Glutamate

Administration of GH causes the redistribution of extracted glutamine nitrogen by the liver from urea into glutamate as measured in the rat hindquarter. Nitrogen balance measurements were made for glutamate and glutamine across the hindquarters of 250-275g hypophysectomized male Sprague-Dawley rats. Recombinant human GH (100μg per 100g per day or vehicle, 0.15 M NaCl), was administered for 3 days prior to the balance measurements. The animals food, but not their drinking solution (5% dextrose) was removed 14 h prior to the balance measurements. Hindquarter plasma flow was estimated by the dilution principle infusing $C^{14}$ labelled paraaminohippurate, 0.01 μCl/min/100g, via the abdominal aorta just above the iliac bifurcation: venous sampling was from a 24 gauge needle fitted to silastic tubing and implanted at the same level. Arterial and venous blood samples were simultaneously drawn and promptly processed for glutamate and glutamine analysis by HPLC. The net uptake or release of nitrogen containing compounds from the plasma flow times arteriovenous concentration differences. The fractional glutamate extraction (FE), was calculated by dividing the net uptake by the arterial load. The administration of GH had the following effects:
1) growth was restored, going from 0.3 ±0.3g/day to 5±1g/day (p=0.01);
2) arterial glutamate was elevated, going from 24 ±2 nmollml to 56 ± 6 nmol/ml (p=0.01);
3) extracted glutamate increased from 10 ±7 to 94 ±15 nmol/min/100g (p=0.01);
4) fractional glutamate extraction increased from 15 ±8 to 46 ±9 percent;
5) muscle glutamate content increased from 573 ±190 to 1314 ± 230 nmollg (p=0.05):
6) muscle glutamine increased from 1,808 ±817 to 3,505 ±530 nmol/g (p=0.10);
7) glutamine synthestase activity of muscle was unchanged.
Enhanced glutamate extraction was associated with a decline in glutamine release from 134 ± 72 to 21 ± 28 nmol/min/100g. The GH administration not only shunts hepatic nitrogen from ureagenesis but enhances recycled glutamate extraction by muscle while diminishing glutamine release.

EXAMPLE 5

Glutamate Determination in Human Subjects

The determination of urinary glutamate can be used to estimate the adequacy of GH action in humans. Children with normal growth and normal GH secretion had urinary glutamate levels on the first voided morning urine of 127 ± 49 nmol/mg creatinine; whereas those with GH deficiency (confirmed by provocative testing) had urinary glutamate levels of 435 ± 162 nmol/mg creatinine (p 0.05). Urinary glutamate levels in the children with confirmed GH deficiency decreased to 53 ± 11 (p 0.05 compared to pretreatment values) when they were placed on exogenous recombinant human GH. In contrast, urinary glutamine to creatinine ratios were not substantially influenced by GH secretory status or exogenous GH administration.

These results demonstrate that the determination of a urinary glutamate to creatinine ratio can be a simple,

EP 0 506 862 B1

rapid test that can: (1) diagnose and/or confirm GH deficiency; (2) measure compliance to GH therapy; (3) measure the adequacy of replacement, and titer the replacement dose, of GH in children or adults with GH deficiency or deficient GH action.

Evaluation of human patients may be accomplished by various methods known to clinical science. These methods are exemplified by the following individual patients evaluated by measuring glutamate, glutamine and alanine in urine.

The patient is brought into the physician's office early in the morning without breakfast, asked to empty his or her bladder and then given a large glass of water. Urine is collected and pooled until flow is relatively constant, about 0 1-2 ml/min and maintained by supplying an equal volume of water to drink. After 30 minutes of relatively stable flow (about 45 minutes in the study from the consumption of the initial water load), the patient is administered 1 mg hGH, or vehicle, by intramuscular injection and consecutive 30 minute urine collections made with volume replacement as above. The entire test should normally last no longer than 2.5 hours. The urine samples were kept on ice and promptly analyzed or frozen. Urine glutamate and glutamine was measured and expressed on the basis of creatinine excreted. A total of seven patients were analyzed and the results presented after reviewing one individual patient's results. The following results in Table 6 are from one GH deficient patient (TS) who was found to have no detectable GH.

### Table 6
### Urine Sample from Patient TS

| Time | GLU* | GLN* | GLN/GLU⁻ | ALA* | ALA/GLU⁻ | GLN/ALA |
|------|------|------|----------|------|----------|---------|
| 10:15 (0 min) (GH 1 mg Admin) | 905 | 1113 | 1.2 | 604 | 0.7 | 1.8 |
| 10:45 (30 min | 300 | 933 | 3.1 | 533 | 1.8 | 1.8 |
| 11:15 (60 min) | 107 | 893 | 8.4 | 513 | 4.8 | 1.7 |
| 11:45 (90 min | 63 | 750 | 11.9 | 500 | 7.9 | 1.5 |
| 12:45 (150 min | 197 | 820 | 4.2 | 426 | 2.2 | 1.9 |
| 1:45 (210 min) | 64 | 516 | 8.7 | 286 | 4.5 | 1.8 |

*nmol/mg creatinine

As clearly seen for patient TS, the response peaks after 1.5 hours. Note that either the GLN/GLU⁻ and ALA/GLU⁻ ratios serve as an adequate index (because the neutral amino acids are not affected by the proton gradient driven transporter $H^+$-$GLU^-$); note also that factoring GLN/ALA give a nearly unvarying ratio. In addition, when the urine pH is promptly evaluated in each of the above urine samples it shows the growth hormone's stimulation of acid secretion. In patient TS pH was as follows: zero min control, pH=6.39, 6.53 (30″), 6.36 (60″) and 6.18 (90″). The urine $NH_4^+$ excretion was also measured for patient TS and it is a good index of the cellular process, 31 (control), 44 (30″), 64 (60″), and 60 (90″). All of these responses to GH in patient TS are consistent with our earlier results in the rat.

Human Urine in Slow Growers

Urine was collected from 7 patients who were slow growers. Urine was collected before GH administration (control) and following a single GH administration (1.0-1.3 mg), urine was collected at 30, 60, 90, 150 and 210 minutes. The amount of glutamate, glutamine and alanine for the 7 patients was determined, averaged and the ratios calculated as illustrated in Table 7.

15

## Table 7

### URINE ANALYSIS IN SLOW GROWERS

| TIME | Glu* | Gln* | Gln/Glu | Ala* | Ala/Glu | Gln/Ala |
|------|------|------|---------|------|---------|---------|
| Control: | 205 ±87 | 749 ±76 | 7.6 | 359 ±64 | 3.4 | 2.3 |
| (GH Administration) | | | | | | |
| 30min | 76 ±45 | 629 ±83 | 14.5 | 251 ±67 | 7.7 | 2.6 |
| 60min | 44 ±17 | 450 ±113 | 27.4 | 282 ±72 | 7.4 | 2.5 |
| 90min | 44 ±14 | 466 ±89 | 24.2 | 235 ±73 | 9.7 | 2.5 |
| 150min | 61 ±27 | 468 ±98 | 20.9 | 192 ±80 | 4.6 | 3.0 |
| 210min | 45 ±10 | 599 ±93 | 16.4 | 181 ±41 | 4.4 | 3.6 |
| GH Treated Average | 54 | 522.4 | 20.68 | 228.2 | 6.76 | 2.84 |

*nmol/mg creatinine, mean ±SEM from 7 patients complete or partial GH deficient, who responded to GH.

These data indicate that two preferred urine criteria are available for determining GH responsiveness in slow growers. The first criteria for slow growers is urinary glutamate per mg creatinine (Glu/creatinine) in the range of 205 187nmol/mg creatinine and a Gln/Glu ratio in the range of 7.6 ±1.9.nmol/mg creatinine These slow grower criteria are about greater than 150 nmol/mg creatinine for Glu/creatinine, and less than 10 nmol/mg creatinine for Gln/Glu.

Normals (non-slow growers) have similar criteria in the range of 88 ±16 and 19 ±4, or about "less than 90" for Glu/creatinine; and "more 15" for Gln/Glu. A patient is considered responsive to GH when the urinary values are about 44 ±17 for Glu and about 27 ±9 for Gln/Glu. It is noted that there seem to be special significance to the Gln/Glu value, since forearm blood exhibits a ratio of less than 17 for slow growers and greater than 27 after GH administration. The muscle Glu value increases from 573 ±190 to 1314 ±203, muscle Gln increases from 1,818 ±817 to 3,505 ±530 nmol/g tissue. However, the Gln/Glu ratio in muscle actually drops because glutamate rises faster than glutamine.

Because a forearm blood sample is frequently drawn, this too can be analyzed using less than 100 ml of plasma. Here are the typical results from an individual slow growing patient

### Table 8

#### Forearm Blood Sample

| Time | Plasma $GLU^-$ | GLN | ALA | $GLN/GLU^-$ | $ALA/GLU^-$ | Urine $GLN/GLU^-$ |
|---|---|---|---|---|---|---|
| 8:00 | 43 | 612 | 640 | 14 | 15 | 6.2 |
| (GH 1 mg Admin) | | | | | | |
| 9:00 | 23 | 649 | 648 | 28 | 28 | 6.3 |
| 10:00 | 25 | 679 | 521 | 27 | 21 | 9.8 |
| 11:00 | 21 | 522 | 451 | 25 | 22 | 10.1 |

Growth hormone increased muscle $GLU^-$ uptake reflected in an elevated $GLN/GLU^-$ ratio. Note that the urine ratio value is a cumulative measurement made over 1 h, while the plasma value is from a single sample at 1 h. As can be seen, the responses are in good agreement. This is consistent with our other studies on glutamate uptake by the rat hindquarters and tissue level.

In summary, there are 3 criteria to evaluate GH response: 1) the absolute rate of glutamate excretion (about>127 ± 49 nmol/mg for GH deficient slow growers and about <53 ± 11 nmol/mg creatinine for GH responsiveness); 2) the ratio of $GLN/GLU^-$ in urine (about >8.6 ± 1.6 for slow growers and less than about 2.0 ± 1.0 for GH responsiveness); and 3) the forearm venous $GLN/GLU^-$ ratio of about <17 for slow growers and about >24 for GH responsiveness). The ratios between 17 and 24 are borderline and additional evaluation and retesting is necessary to determine GH responsiveness. Interestingly, the forearm venous blood ratio is quite predictive for a positive GH response since only 10-20 percent of the glutamate flowing through this site is taken up, in contrast to 50 percent with GH. Urine glutamate, on the other hand, is normally efficiently removed by the kidney (97%), and GH may increase this uptake to 99%. This difference may seem a small increment in terms of absolute uptake, however it is highly reflective of the powerful stimulation of acid secretion by GH.

EXAMPLE 6

#### Urinary Glutamate/Creatinine Ratio and Its Use In GH Deficiency

To evaluate whether glutamate/creatinine ratios values could be used to predict possible growth deficiency, urine was collected from 10 children with documented growth hormone deficiency (GHD) and 15 non-GH deficient children. Prior studies we conducted of randomly collected, pre-acidified urine suggested that the determination of urinary glutamate to creatinine ratio could be useful to predict GHD and to follow the course of GH treatment.

The GHD patient sample consisted of random non-acidified urine from 10 children with documented GHD (height at least 2 SD's below mean for age and 2 GH-provocative tests with GH <10ng/mL) to blind analysis. According to the present invention, we would have expected these children to have demonstrated a high urinary glutamate/creatinine ratio. Eight of the ten (80%) demonstrated urinary ratios of greater than 75 nmol glutamate/g creatinine.

Fifteen random non-acidified urine samples were submitted from non-GHD children (height within 2 SD's for age or stimulated GH>10ng/mL) for blinded analysis. Nine out of the 15 (60%) had urinary values of less than 75 nmol glutamate/g creatinine. Therefore, from this patient group we estimate that this test has a sensitivity to confirm GHD of approximately 80% with a specificity of approximately 60%. Specificity is a measure of ability of the assay to distinguish normal growers from slow growers. Sensitivity is a measure of ability to detect GHD. This sensitivity will be improved by acidifying the urine, thereby reducing the conversion of glutamate to glutamine or to other metabolites in alkaline urine.

These non-acidified urine values compare favorably with the current best diagnosis method for GHD-growth hormone provocative testing, which has a low sensitivity and specificity. A recent abstract (Marin G. et al., "Responses to 4 GH provocative tests in normal children and adolescents", American Pediatric Society/Society for Pediatric Research Annual Meeting, 1990) suggested that approximately 33% of normal children fail to show a response of 7 ng/ml to 3 GH provocative tests (arginine, insulin, clonidine). Thus, the specificity of the current best test for GHD may only be 60-70%. With pre-acidified urine collection, the urinary glutamate/creatinine determination surpasses 60% specificity.

## Claims

1. A method for diagnosing deficiency in a hormonal agent of nitrogen balance (HANB) in a patient comprising:
   a) providing a sample body fluid or tissue;
   b) quantitating the molar concentration of glutamate and a second compound selected from glutamine, alanine, urea or creatinine in said sample;
   c) determining the molar ratio of said glutamate to said second compound;
   d) comparing said molar ratio with a standard molar ratio found in a sample of patients known not to have a deficiency in a HANB and if said molar ratio is statistically different from said standard molar ratio, then the diagnosis is a deficiency in a hormonal agent of nitrogen balance

2. The method according to claim 1 wherein said body fluid is selected from the following: serum, blood, urine, sputum, mucus, amniotic fluid, perspiration, tears, saliva, spinal fluid, milk, semen or plasma.

3. The method according to claim 1 wherein said HANB is growth hormone, insulin-like growth factor-1, growth hormone binding protein or an insulin-like growth factor binding protein.

4. The method according to claim 2 wherein said bodily fluid is urine, said second compound is glutamine and the standard molar ratio for glutamine/glutamate is less than 10

5. The method according to claim 2 wherein said bodily fluid is urine, said second compound is arginine and the standard molar ratio for alanine/glutamate is less than 5

6. The method according to claim 2 wherein said bodily fluid is urine, said second compound is creatinine and the standard molar ratio is replaced by a standard mole/weight ratio for glutamate/creatinine is greater than 150 nmol/mg creatinine.

7. A method for determining the dosage of a hormonal agent of nitrogen balance (HANB) in a patient comprising:
   (a) obtaining a sample of urine from the patient prior to the administration of the HANB;
   (b) determining the ratio of glutamate to a second compound selected from the group of glutamine, alanine, urea and creatinine, or the reciprocal thereof, in the urine sample of step (a);
   (c) administering a test dose of HANB to the patient:
   (d) collecting a sample of urine from the patient;
   (e) determining the ratio of glutamate to said second compound selected from the group of glutamine, alanine, urea and creatinine, or the reciprocal thereof, in the urine sample of part (d), provided that the ratio is calculated on the same basis or reciprocal as was used in part (b);and
   (f) determining the difference between the ratio of step (b) and step (e), and if the difference is statistically significant, adjusting the HANB dosage up or down as appropriate until the difference between step (b) and step (e) is not statistically significant.

8. The method according to claim 7 wherein the ratio is that of glutamate to glutamine.

9. The method according to claim 8 wherein if the ratio determined in step (b) substantially exceeds that which is determined in step (d) then increasing the patients HANB dosage.

10. The method according to claim 8 wherein if the ratio determined in step (b) is substantially less than that which is determined in step (d), then decreasing the patients HANB dosage.

11. The method according to claim 8 wherein if the ratio determined in step (b) is substantially the same as that which is determined in step (d), then maintaining the patient's HANB dosage.

12. The method according to claim 8 wherein the HANB is selected from human growth hormone, insulin-like growth factor-1, growth hormone binding protein or an insulin-like growth factor binding protein.

13. The method according to claim 1 wherein said HANB is human growth hormone or human growth hormone plus growth hormone binding protein.

14. The method of claim 7 wherein said patient is younger than 20 years of age.

15. The method according to claim 14 wherein if said ratio increases, then administering to the patient an increased dosage of HANB.

16. The method according to claim 14 wherein if said ratio decreases, then administering to the patient a decreased dosage of HANB

17. A method of monitoring the metabolic condition of a patient receiving a hormonal agent of nitrogen balance (HANB) comprising:
    (a) obtaining a sample of urine from the patient prior to the therapeutic administration of the HANB;
    (b) determining the ratio of glutamate to a second compound selected from the group of glutamine, alanine, urea and creatinine, or the reciprocal thereof, in the urine sample of step (a);
    (c) administering the therapeutic dose of HANB to the patient:
    (d) collecting a sample of urine from the patient; and,
    (e) determining the ratio of glutamate to said second compound selected from the group of glutamine, alanine, urea and creatinine, or the reciprocal thereof, in the urine sample of part (d), provided that the ratio is calculated on the same basis or reciprocal as was used in part (b); and
    (f) determining the difference between the ratio of step (b) and step (e), and if the difference is statistically significant, adjusting the HANB dosage up or down as appropriate until the difference between step (b) and step (e) is not statistically significant.

18. The method according to claim 17 wherein the ratio is that of glutamate to glutamine and the HANB is selected from growth hormone or insulin-like growth factor-1.

19. The method according to claim 18 wherein said growth hormone is human growth hormone or human growth hormone plus growth hormone binding protein.

20. The method according to claim 18 wherein said insulin-like growth factor-1 is human insulin-like growth factor-1 or human insulin-like growth factor-1 plus insulin-like growth factor binding protein.

21. The method according to claim 1 wherein said deficiency in a HANB is diagnosed in said patient following treatment with one of the following : surgery, radiation, chemotherapy, antibiotics, hormone agonists, hormone antagonists, hormone inhibitors, antibodies, or nutritional supplements.

22. A method for diagnosing deficiency in a hormonal agent of nitrogen balance (HANB) in a patient comprising:
    a) providing a urine sample;
    b) determining whether the concentration of glutamate in said sample is greater than 150 nmol/mg creatinine.

23. A method for diagnosing deficiency in a hormonal agent of nitrogen balance (HANB) in a patient comprising:
    a) providing a urine sample;
    b) quantitating the concentration of glutamate and glutamine in said sample;
    c) determining whether the ratio of said glutamine divided by said glutamate or glutamic acid is less than 10

24. Glutamate for use in promoting growth in a mammal deficient in a hormonal agent of nitrogen balance (HANB).

25. Use of glutamate in the manufacture of a composition for administration to a mammal deficient in a hormonal agent of nitrogen balance (HANB) for promoting growth.

26. Use according to claim 25 wherein said composition is for administration by a route selected from the following: oral, topical, parenteral injection such as intramuscular injection, subcutaneous injection or intravenous injection; suppository, or inhalation.

27. Use according to claim 25 or 26 wherein said administration is in a dosage of glutamate of 1.0 to 800 mg/kg/day.

28. Use according to claim 25 or 26 wherein said administration is in a dosage of glutamate of 10 to 400 mg/kg/day.

29. Use according to claim 25 or 26 wherein said administration is in a dosage of glutamate of 50 to 150 mg/kg/day.

**Patentansprüche**

1. Verfahren zum Diagnostizieren eines Mangels an einem Hormonalwirkstoff des Stickstoffgleichgewichts (HANB) bei einem Patienten, umfassend:
   a) Abnehmen einer Körperfluid- oder Gewebsprobe;
   b) Quantifizieren der molaren Konzentration von Glutamat und einer zweiten Verbindung, ausgewählt aus Glutamin, Alanin, Harnstoff oder Kreatinin, in dieser Probe;
   c) Bestimmen des Molverhältnisses des Glutamats zu der zweiten Verbindung;
   d) Vergleichen dieses Molverhältnisses mit einem Normmolverhältnis in einer Probe von Patienten, die bekannterweise nicht unter einem Mangel an HANB leiden, und falls dieses Molverhältnis statistisch verschieden von diesem Normmolverhältnis ist, lautet die Diagnose: Mangel an einem Hormonalwirkstoff des Stickstoffgleichgewichts.

2. Verfahren nach Anspruch 1, worin dieses Körperfluid aus den folgenden ausgewählt wird: Serum, Blut, Harn, Sputum, Schleim, amniotisches Fluid, Schweiß, Tränen, Speichel, Rückenmarksfluid, Milch, Samen oder Plasma.

3. Verfahren nach Anspruch 1, worin dieser HANB Wachstumshormon, insulinähnlicher Wachstumsfaktor 1, proteinbindendes Wachstumshormon oder proteinbindender insulinähnlicher Wachstumsfaktor ist.

4. Verfahren nach Anspruch 2, worin das Körperfluid Harn, die zweite Verbindung Glutamin und das Normmolverhältnis für Glutamin/Glutamat weniger als 10 ist.

5. Verfahren nach Anspruch 2, worin das Körperfluid Harn, die zweite Verbindung Arginin und das Normmolverhältnis für Alanin/Glutamat weniger als 5 ist.

6. Verfahren nach Anspruch 2, worin das Körperfluid Harn, die zweite Verbindung Kreatinin ist und das Normmolverhältnis durch ein Normmol/Gewichtsverhältnis ersetzt wird, das für Glutamat/Kreatinin größer als 150 nMol/mg Kreatinin ist.

7. Verfahren zur Bestimmung der Dosierung eines Hormonalwirkstoffs des Stickstoffgleichgewichts (HANB) bei einem Patienten, umfassend:
   (a) Abnehmen einer Harnprobe des Patienten vor der Verabreichung des HANB;
   (b) Bestimmen des Verhältnisses oder dessen Reziprokwerts von Glutamat zu einer zweiten Verbindung, die aus der Gruppe aus Glutamin, Alanin, Harnstoff und Kreatinin ausgewählt ist, in der Harnprobe aus Schritt (a);
   (c) Verabreichen einer Testdosis von HANB an den Patienten;
   (d) Abnehmen einer Harnprobe des Patienten;
   (e) Bestimmen des Verhältnisses oder dessen Reziprokwerts von Glutamat zur zweiten Verbindung, die aus der Gruppe aus Glutamin, Alanin, Harnstoff und Kreatinin ausgewählt ist, in der Harnprobe aus Teil (d), mit der Maßgabe, daß die Berechnung des Verhältnisses oder des Reziprokwerts auf derselben Basis erfolgt wie in Teil (b); und
   (f) Bestimmen der Differenz der Verhältnisse aus Schritt (b) und (e), und, falls die Differenz statistisch signifikant ist, Einstellen (Erhöhen oder Verringern) der geeigneten HANB-Dosierung, bis die Differenz zwischen Schritt (b) und (e) statistisch nicht signifikant ist.

8. Verfahren nach Anspruch 7, worin das Verhältnis das von Glutamat zu Glutamin ist.

9. Verfahren nach Anspruch 8, worin die HANB-Dosierung an den Patienten gesteigert wird, falls das in Schritt (b) bestimmte Verhältnis das in Schritt (d) bestimmte beträchtlich überschreitet.

10. Verfahren nach Anspruch 8, worin die HANB-Dosierung an den Patienten verringert wird, falls das in Schritt (b) bestimmte Verhältnis wesentlich geringer ist als das in Schritt (d) bestimmte.

11. Verfahren nach Anspruch 8, worin die HANB-Dosierung an den Patienten beibehalten wird, falls das in Schritt (b) bestimmte Verhältnis im wesentlichen gleich dem in Schritt (d) bestimmten ist.

12. Verfahren nach Anspruch 8, worin der HANB aus menschlichem Wachstumshormon, insulinähnlichem Wachstumsfaktor 1, proteinbindendem Wachstumshormon oder proteinbindendem insulinähnichem Wachstumsfaktor ausgewählt wird.

13. Verfahren nach Anspruch 1, worin der HANB menschliches Wachstumshormon oder menschliches Wachstumshormon plus proteinbindendes Wachstumshormon ist.

14. Verfahren nach Anspruch 7, worin der genannte Patient jünger als 20 Jahre ist.

15. Verfahren nach Anspruch 14, worin dem Patienten eine erhöhte Dosis von HANB verabreicht wird, falls dieses Verhältnis steigt.

16. Verfahren nach Anspruch 14, worin dem Patienten eine verringerte Dosis von HANB verabreicht wird, falls dieses Verhältnis sinkt.

17. Verfahren zur Überwachung des Stoffwechselzustands eines Patienten, der einen Hormonalwirkstoff des Stickstoffgleichgewichts (HANB) erhält, umfassend:
    (a) Abnehmen einer Harnprobe des Patienten vor der therapeutischen Verabreichung des HANB;
    (b) Bestimmen des Verhältnisses oder des Reziprokwerts von Glutamat zu einer zweiten Verbindung, die aus der Gruppe aus Glutamin, Alanin, Harnstoff und Kreatinin ausgewählt ist, in der Harnprobe aus Schritt (a);
    (c) Verabreichen der therapeutischen Dosis von HANB an den Patienten;
    (d) Abnehmen einer Harnprobe des Patienten; und
    (e) Bestimmen des Verhältnisses oder dessen Reziprokwerts von Glutamat zur zweiten Verbindung, die aus der Gruppe aus Glutamin, Alanin, Harnstoff und Kreatinin ausgewählt ist, in der Harnprobe aus Teil (d), mit der Maßgabe, daß die Berechnung des Verhältnisses oder des Reziprokwerts auf derselben Basis erfolgt wie in Teil (b); und
    (f) Bestimmen der Differenz der Verhältnisse aus Schritt (b) und (e), und, falls die Differenz statistisch signifikant ist, Einstellen (Erhöhen oder Verringern) der geeigneten HANB-Dosierung, bis die Differenz zwischen Schritt (b) und (e) statistisch nicht signifikant ist.

18. Verfahren nach Anspruch 17, worin das Verhältnis das von Glutamat zu Glutamin ist und der HANB aus Wachstumshormon oder insulinähnlichem Wachstumsfaktor 1 ausgewählt wird.

19. Verfahren nach Anspruch 18, worin das Wachstumshormon menschliches Wachstumshormon oder menschliches Wachstumshormon plus proteinbindendes Wachstumshormon ist.

20. Verfahren nach Anspruch 18, worin der insulinähnliche Wachstumsfaktor 1 menschlicher insulinähnlicher Wachstumsfaktor 1 oder menschlicher insulinähnlicher Wachstumsfaktor 1 plus proteinbindender insulinähnicher Wachstumsfaktor ist.

21. Verfahren nach Anspruch 1, worin der Mangel an HANB bei diesem Patienten diagnostiziert und von einer der folgenden Behandlungen gefolgt wird: chirurgische Behandlung, Bestrahlung, Chemotherapie, Antibiotika, Hormonagonisten, Hormonantagonisten, Hormoninhibitoren, Antikörper oder Ernährungszusätze.

22. Verfahren zum Diagnostizieren eines Mangels an einem Hormonalwirkstoff des Stickstoffgleichgewichts (HANB) bei einem Patienten, umfassend:
    a) Abnehmen einer Harnprobe;

b) Bestimmen, ob die Konzentration von Glutamat in dieser Probe höher als 150 nMol/mg Kreatinin ist.

23. Verfahren zum Diagnostizieren eines Mangels an einem Hormonalwirkstoff des Stickstoffgleichgewichts (HANB) bei einem Patienten, umfassend:
    a) Abnehmen einer Harnprobe;
    b) Quantifizieren der Konzentration von Glutamat und Glutamin in dieser Probe;
    c) Bestimmen, ob das Verhältnis des Glutamins dividiert durch dieses Glutamat oder Glutaminsäure weniger als 10 beträgt.

24. Glutamat zur Verwendung beim Fördern des Wachstums bei einem Säuger mit einem Mangel an einem Hormonalwirkstoff des Stickstoffgleichgewichts (HANB).

25. Verwendung von Glutamat bei der Herstellung einer Zusammensetzung zur Verabreichung an einen Säuger mit einem Mangel an einem Hormonalwirkstoff des Stickstoffgleichgewichts (HANB), um das Wachstum zu fördern.

26. Verwendung nach Anspruch 25, worin die Zusammensetzung zur Verabreichung auf einem der folgenden Wege dient: oral, topisch, Parenteralinjektion, wie z.B. intramuskuläre Injektion, subkutane Injektion oder intravenöse Injektion; Zäpfchen oder Inhalation.

27. Verwendung nach Anspruch 25 oder 26, worin die Verabreichung mit einer Dosierung an Glutamat von 1,0 bis 800 mg/kg/Tag erfolgt.

28. Verwendung nach Anspruch 25 oder 26, worin die Verabreichung mit einer Dosierung an Glutamat von 10 bis 400 mg/kg/Tag erfolgt.

29. Verwendung nach Anspruch 25 oder 26, worin die Verabreichung mit einer Dosierung an Glutamat von 50 bis 150 mg/kg/Tag erfolgt.

**Revendications**

1. Méthode pour diagnostiquer une déficience en un agent hormonal du bilan azoté (AHBA) chez un patient, comprenant :
    a) le prélèvement d'un échantillon de liquide biologique ou de tissu ;
    b) la quantification de la concentration molaire de glutamate et d'un second composé choisi entre la glutamine, l'alanine, l'urée et la créatinine dans ledit échantillon ;
    c) la détermination du rapport molaire dudit glutamate audit second composé ;
    d) la comparaison dudit rapport molaire à un rapport molaire de référence obtenu avec un échantillon provenant de patients connus pour ne pas présenter une déficience en un AHBA et, si ledit rapport molaire est statistiquement différent dudit rapport molaire de référence, l'obtention d'un diagnostic de déficience en un agent hormonal du bilan azoté.

2. Méthode suivant la revendication 1, dans laquelle le liquide biologique est choisi entre les suivants : le sérum, le sang, l'urine, des expectorations des mucosités, le liquide amniotique, la sueur, les larmes, la salive, le liquide rachidien, le lait, le sperme ou le plasma.

3. Procédé suivant la revendication 1, dans lequel le AHBA est l'hormone de croissance, le facteur de croissance 1 analogue à l'insuline, la protéine fixant l'hormone de croissance ou une protéine de liaison à un facteur de croissance analogue à l'insuline.

4. Méthode suivant la revendication 2, dans laquelle le liquide biologique est l'urine, le second composé est la glutamine et le rapport molaire de référence, glutamine/glutamate est inférieur à 10.

5. Méthode suivant la revendication 2, dans laquelle le liquide biologique est l'urine, le second composé est l'arginine et le rapport molaire de référence alanine/glutamate est inférieur à 5.

6. Méthode suivant la revendication 2, dans laquelle le liquide biologique est l'urine, le second composé est la créatinine et le rapport molaire de référence est remplacé par un rapport de référence en moles/poids

EP 0 506 862 B1

glutamate/créatinine supérieur à 150 nmoles/mg de créatinine.

7. Méthode pour déterminer la dose d'un agent hormonal du bilan azoté (AHBA) chez un patient, comprenant :
(a) l'obtention d'un échantillon d'urine du patient avant administration du AHBA ;
(b) la détermination du rapport du glutamate à un second composé choisi dans le groupe comprenant la glutamine, l'alanine, l'urée et la créatinine, ou bien de l'inverse de ce rapport, dans l'échantillon d'urine de l'étape (a) ;
(c) l'administration d'une dose d'essai de AHBA au patient ;
(d) le prélèvement d'un échantillon d'urine du patient ;
(e) la détermination du rapport du glutamate audit second composé choisi dans le groupe consistant en la glutamine, l'alanine, l'urée et la créatinine, ou bien de l'inverse de ce rapport, dans l'échantillon d'urine de la partie (d), sous réserve que le rapport soit calculé sur la même base ou soit l'inverse de celui qui a été utilisé dans la partie (b) ; et
(f) la détermination de la différence entre le rapport de l'étape (b) et le rapport de l'étape (e) et, si la différence est statistiquement significative, l'ajustement de la dose de AHBA par augmentation ou diminution de la manière appropriée jusqu'à ce que la différence entre l'étape (b) et l'étape (e) ne soit pas statistiquement significative.

8. Méthode suivant la revendication 7, dans laquelle le rapport est le rapport du glutamate à la glutamine.

9. Méthode suivant la revendication 8, dans laquelle, si le rapport déterminé dans l'étape (b) excède notablement celui qui est déterminé dans l'étape (d), la dose de AHBA fournie au patient est alors accrue.

10. Méthode suivant la revendication 8, dans laquelle, si le rapport déterminé dans l'étape (b) est notablement inférieur à celui qui est déterminé dans l'étape (d), la dose de AHBA fournie au patient est alors maintenue.

11. Méthode suivant la revendication 8, dans laquelle, si le rapport déterminé dans l'étape (b) est pratiquement identique à celui qui est déterminé dans l'étape (d), la dose de AHBA fournie au patient est alors réduite.

12. Méthode suivant la revendication 8, dans laquelle, le AHBA est choisi entre l'hormone de croissance humaine, le facteur de croissance 1 analogue à l'insuline, une protéine fixant l'hormone de croissance ou une protéine fixant un facteur de croissance analogue à l'insuline.

13. Méthode suivant la revendication 1, dans laquelle le AHBA est l'hormone de croissance humaine ou l'hormone de croissance humaine plus la protéine fixant l'hormone de croissance.

14. Méthode suivant la revendication 7, dans laquelle le patient est âgé de moins de 20 ans.

15. Méthode suivant la revendication 14, dans laquelle, si le rapport augmente, une dose accrue de AHBA est alors administrée au patient.

16. Méthode suivant la revendication 14, dans laquelle, si le rapport diminue, une dose réduite de AHBA est alors administrée au patient.

17. Méthode pour contrôler l'état métabolique d'un patient recevant un agent hormonal du bilan azoté (AHBA), comprenant :
(a) l'obtention d'un échantillon d'urine du patient avant l'administration thérapeutique du AHBA ;
(b) la détermination du rapport du glutamate à un second composé choisi dans le groupe consistant en la glutamine, l'alanine, l'urée et la créatinine, ou de l'inverse de ce rapport, dans l'échantillon d'urine de l'étape (a) ;
(c) l'administration de la dose thérapeutique de AHBA au patient ;
(d) le prélèvement d'un échantillon d'urine du patient ; et
(e) la détermination du rapport du glutamate au second composé choisi dans le groupe consistant en la glutamine, l'alanine, l'urée et la créatinine, ou de l'inverse de ce rapport, dans l'échantillon d'urine de la partie (d), sous réserve que le rapport ou son inverse soit calculé sur la même base que celui utilisé dans la partie (b) ; et
(f) la détermination de la différence entre le rapport de l'étape (b) et le rapport de l'étape (e) et, si la

23

différence est statistiquement significative, l'ajustement de la dose de AHBA par augmentation ou diminution de la manière appropriée jusqu'à ce que la différence entre l'étape (b) et l'étape (e) ne soit pas statistiquement significative.

18. Méthode suivant la revendication 17, dans laquelle le rapport est le rapport du glutamate à la glutamine et le AHBA est choisi entre l'hormone de croissance et le facteur de croissance 1 analogue à l'insuline.

19. Méthode suivant la revendication 18, dans laquelle l'hormone de croissance est l'hormone de croissance humaine ou l'hormone de croissance humaine plus la protéine fixant l'hormone de croissance.

20. Méthode suivant la revendication 18, dans laquelle le facteur de croissance 1 analogue à l'insuline est le facteur de croissance 1 humain analogue à l'insuline ou le facteur de croissance 1 humain analogue à l'insuline plus la protéine fixant le facteur de croissance analogue à l'insuline.

21. Méthode suivant la revendication 1, dans laquelle la déficience en un AHBA est diagnostiquée chez le patient après un des traitements suivants : une intervention chirurgicale, une irradiation, une chimiothérapie, l'administration d'antibiotiques, l'administration d'agonistes d'hormones, l'administration d'antagonistes d'hormones, l'administration d' inhibiteurs d'hormones, l'administration d'anticorps ou l'administration d'agents de supplémentation nutritionnelle.

22. Méthode pour diagnostiquer une déficience en un agent hormonal du bilan azoté (AHBA) chez un patient comprenant :
   a) le prélèvement d'un échantillon d'urine ;
   b) l'étape consistant à déterminer si la concentration de glutamate dans ledit échantillon est supérieure à 150 nmoles/mg de créatinine.

23. Méthode pour diagnostiquer une déficience en un agent hormonal du bilan azoté (AHBA) chez un patient, comprenant :
   a) le prélèvement d'un échantillon d'urine ;
   b) la quantification de la concentration de glutamate et de glutamine dans ledit échantillon ;
   c) l'étape consistant à déterminer si le rapport de la glutamine au glutamate ou à l'acide glutamique est inférieur à 10.

24. Glutamate destiné à être utilisé pour activer la croissance d'un mammifère présentant une déficience en un agent hormonal du bilan azoté (AHBA).

25. Utilisation de glutamate dans la production d'une composition destinée à être administrée à un mammifère présentant une déficience en un agent hormonal du bilan azoté (AHBA) pour activer la croissance.

26. Utilisation suivant la revendication 25, dans laquelle la composition est destinée à l'administration par une voie choisie entre les suivantes : la voie orale, la voie locale, l'injection parentérale telle que l'injection intramusculaire, l'injection sous-cutanée ou l'injection intraveineuse ; l'administration d'un suppositoire, ou l'inhalation.

27. Utilisation suivant la revendication 25 ou 26, dans laquelle l'administration est effectuée à une dose de glutamate de 1,0 à 800 mg/kg/jour.

28. Utilisation suivant la revendication 25 ou 26, dans laquelle l'administration est effectuée à une dose de glutamate de 10 à 400 mg/kg/jour.

29. Utilisation suivant la revendication 25 ou 26, dans laquelle l'administration est effectuée à une dose de glutamate de 50 à 150 mg/kg/jour.

FIG. I